(19) Europäisches Patentamt
European Patent Office
Office européen des brevets

(11) EP 1 481 675 A1

(12) EUROPEAN PATENT APPLICATION
published in accordance with Art. 158(3) EPC

(43) Date of publication:
**01.12.2004 Bulletin 2004/49**

(21) Application number: **03743564.1**

(22) Date of filing: **03.03.2003**

(51) Int Cl.[7]: **A61K 31/23**, A61K 31/165, A61K 31/20, A61K 31/355, A61K 31/426, A61K 31/4415, A61K 31/661, A61K 33/06, A61K 33/26, A61K 33/30, A61K 35/78, A61K 38/16, A61P 3/04, A61P 3/06, A61P 3/10, A61P 9/00, A61P 43/00, A23D 9/00

(86) International application number:
**PCT/JP2003/002442**

(87) International publication number:
**WO 2003/074043 (12.09.2003 Gazette 2003/37)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **04.03.2002 JP 2002058097**
**05.09.2002 JP 2002260509**

(71) Applicant: **The Nisshin Oillio Group, Ltd.**
**Tokyo 104-8285 (JP)**

(72) Inventors:
- **SHINOHARA, H., c/o The Nisshin Oillio,Ltd.**
  **Yokosuka-shi, Kanagawa 239-0832 (JP)**
- **NOGUCHI, O., c/o The Nisshin Oillio, Ltd.**
  **Yokosuka-shi, Kanagawa 239-0832 (JP)**
- **ASAMI, F.**
  **Yokohama-shi, Kanagawa 236-0057 (JP)**
- **SHIMADA, H., c/o The Nisshin Oillio, Ltd.**
  **Yokosuka-shi, Kanagawa 239-083 (JP)**
- **INUI, Toshiyuki**
  **Ebina-shi, Kanagawa 243-0411 (JP)**

(74) Representative: **Merkle, Gebhard**
**TER MEER STEINMEISTER & PARTNER GbR,**
**Patentanwälte,**
**Mauerkircherstrasse 45**
**81679 München (DE)**

# (54) BODY TEMPERATURE ELEVATING AGENTS

(57) The present invention provides body temperature elevating agents and foods and drinks for body temperature elevation. The invention relates to the body temperature elevating agent and food and drink for the body temperature elevation, which is comprised of medium chain fatty acid and/or glycerin fatty acid ester including the medium chain fatty acid, preferably medium chain fatty acid triglycerin as an active ingredient(s).

Fig. 1A

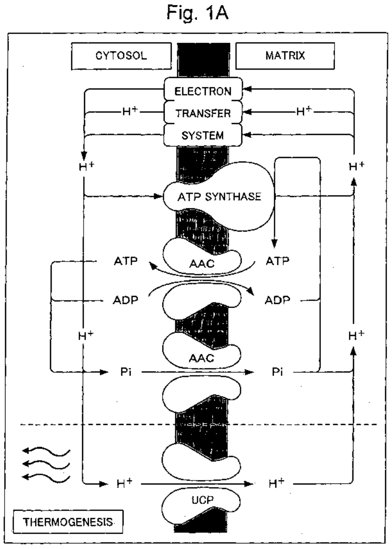


EP 1 481 675 A1

Fig. 1B

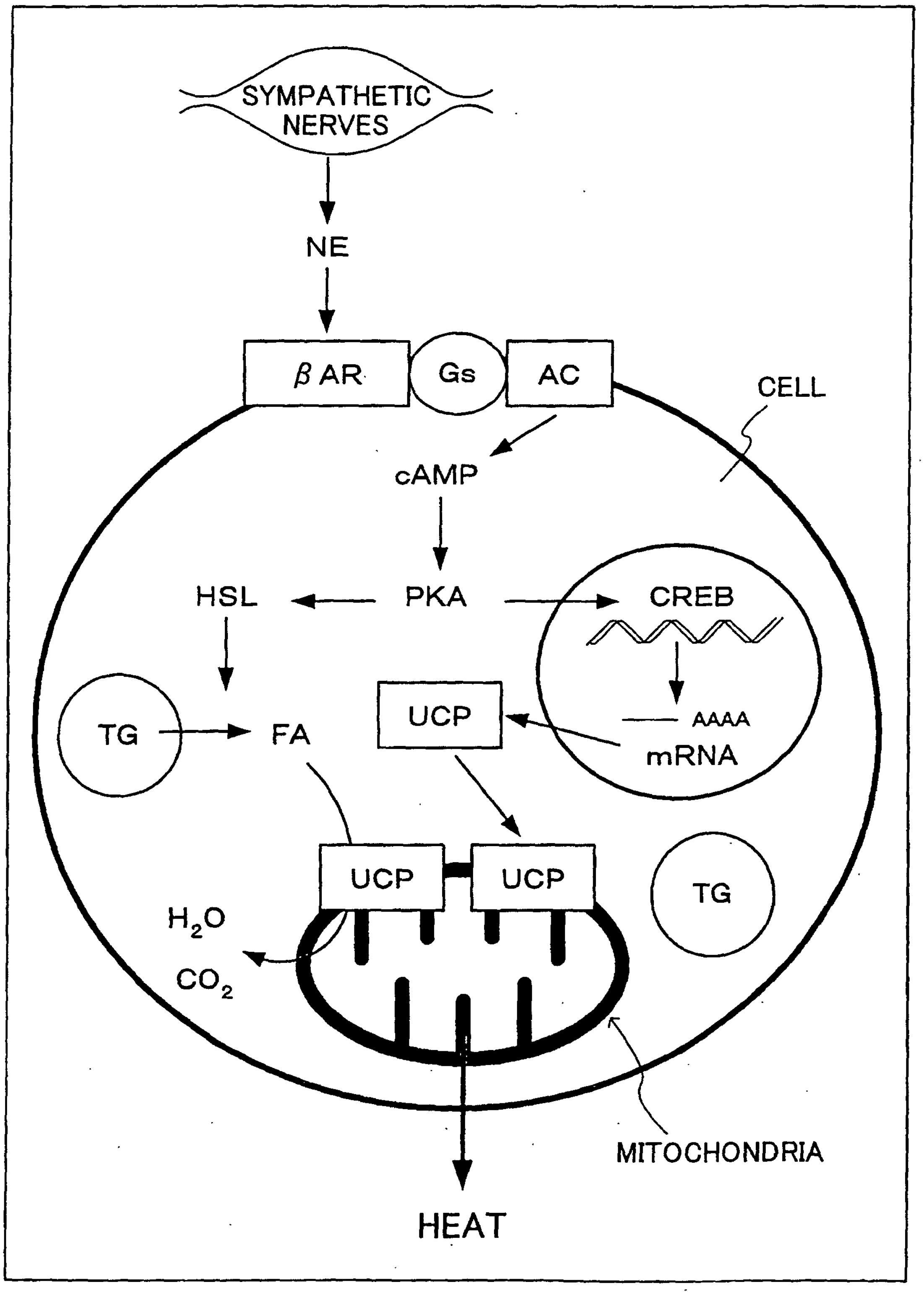
SYMPATHETIC NERVES
NE
β AR
Gs
AC
CELL
cAMP
PKA
HSL
CREB
TG
FA
UCP
AAAA
mRNA
UCP
UCP
TG
$H_2O$
$CO_2$
MITOCHONDRIA
HEAT

## Description

Field of the Art

The present invention relates to a body temperature elevating agent which is comprised of a medium chain fatty acid and/or a glycerin fatty acid ester containing the medium chain fatty acid as an active ingredient. More particularly, the present invention relates to a body temperature elevating agent which is comprised of a medium chain fatty acid and/or a glycerin fatty acid ester containing the medium chain fatty acid having an action to specifically facilitate expression of uncoupling protein (hereinafter abbreviated as "UCP") possessing functions such as prevention of obesity by consuming excessive energy and producing heat, as an active ingredient, and also relates to a fat and oil composition and foods and drinks.

Background Art

Means to warm up a body cooled by artificial cooling in the summer season or in the winter season include methods such as facilitating blood circulation by massage of limbs or the use of commercially available heat insulating materials/agents and incorporating heat in the body by the ingestion of warm foods and drinks. Also, massage agents used therefor have been developed. However, by these methods, immediately after use, the blood circulation is temporarily facilitated, and warming sensation of the body is obtained, however, their effects are not sustained. Additionally, recently along with aging of the population, patients who complain about symptoms such as coldness, numbness and pain due to peripheral blood flow disorder have increased. Thus, development of medicaments has been earnestly desired which have sufficient therapeutic effects regarding such symptoms at local positions of disorder sites, less systemic effects, high safety even for the elderly, and can treat symptoms such as coldness, numbness and pain caused by peripheral blood flow disorder.

An energy metabolic regulation system of a living body is composed of an ingestion regulation system and an energy consumption regulation system. In these, the energy consumption regulation system can be divided into an energy consumption used for basic metabolism for life support and the remaining energy consumption. As the latter, the main feature is non-shivering thermogenesis, of which functional significance includes maintenance of body temperature and consumption of excessively ingested energy.

It is known that uncoupling protein (hereinafter referred to as "UCP") is involved in an elicitation mechanism of this nonshivering thermogenesis. UCP is a special protein present in brown adipose tissues of rodents and hibernants, and is known as a thermogenic molecule which uncouples oxidative phosphorylation in mitochondria to scatter oxidation energy asheat. Recently it has been demonstrated that UCP is expressed in many tissues and cells including skeletal muscles in humans (e.g., see Saito M., Ohashi A., Nippon Yakurigaku Zasshi, 118:327-333, 2001). Therefore, it has become a critical problem to search for a substance which specifically facilitates UCP.

Meanwhile, it is known that medium chain fatty acids are easy to be made into energy and thus accumulation of body fat is low (e.g., see J. Lipid Res., 37:708-726, 1996; Saito M., Ohashi A., Nippon Yakurigaku Zasshi, 118: 327-333, 2001). Also, it has been reported that the accumulation of body fat is low in a fat and oil composition comprising 31% or more by mass of triacylglycerol containing two medium chain fatty acid residues in a molecule in fat and oil composition ingredients composed of diacylglycerol and triacylglycerol (e.g., see Japanese Published Unexamined Patent Application No. 4-300826A, Japanese Published Unexamined Patent Application No. 8-60181A and Japanese Published Unexamined Patent Application No. 10-176181A). Furthermore, it has been reported that diacylglycerol and conjugated linoleic acid have an action to specifically facilitate the expression of UCP (e.g., see JP 2001-64171 and JP 2001-81026). However, it has not been known that medium chain triacylglycerol specifically facilitates UCP and has a body temperature elevating function by diet induced body thermogenesis, and the like.

The invention provides body temperature elevating agents having a function to elevate body temperature, foods and drinks for body temperature elevation, and rawmaterial agents thereof, and provides agents for poor blood circulation and agents for women to which a body temperature elevating function and other functions are added.

Disclosure of the Invention

The present inventors have found that a medium chain fatty acid and/or a glycerin fatty acid ester including the medium chain fatty acid, particularlyamediumchain fatty acid glycerin fatty acid triester (hereinafter sometimes referred to as "medium chain triacylglycerol," "MCT") has a body temperature elevating function, and have achieved the invention.

The invention comprises the following contents.

(1) A body temperature elevating agent which is comprised of a medium chain fatty acid and/or a glycerin fatty

acid ester including the medium chain fatty acid as an active ingredient.

(2) The body temperature elevating agent according to (1), wherein a carbon number in fatty acid residues of the above medium chain fatty acid and/or glycerin fatty acid ester including the medium chain fatty acid is from 6 to 12.

(3) The body temperature elevating agent according to (1) or (2), wherein the above fatty acid which is comprised of one or two or more selected from the group consisting of caproic acid, caprylic acid, capric acid, and lauric acid an active ingredient.

(4) A body temperature elevating agent which is comprised of a medium chain fatty acid glycerin fatty acid triester as an active ingredient.

(5) The body temperature elevating agent according to (4), wherein a carbon number in fatty acid residues of the above medium chain fatty acid and/or glycerin fatty acid ester including the medium chain fatty acid is from 6 to 12.

(6) The body temperature elevating agent according to (4) or (5), wherein the above fatty acid which is comprised of one or two or more selected from the group consisting of caproic acid, caprylic acid, capric acid, and lauric acid as an active ingredient.

(7) The body temperature elevating agent according to any one of (1) to (6), wherein further containing one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

(8) The body temperature elevating agent according to any one of (1) to (7), wherein further containing one or two or more selecting from the group consisting of vitamin B group, vitamin E, lecithin, minerals and herbs.

(9) An agent for poor blood circulation, wherein containing (A) a medium chain fatty acid and/or a glycerin fatty acid ester including the medium chain fatty acid, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

(10) An agent for poor blood circulation, wherein containing (A) a medium chain triacylglycerol, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

(11) An agent for women wherein containing (A) a medium chain fatty acid and/or a glycerin fatty acid ester including the medium chain fatty acid, (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides, and (C) one or two or more selected from the group consisting of γ-linolenic acid, dihome-γ-linolenic acid, calcium, magnesium, zinc, iron, vitamin B6, vitamin E, St. John's wort and extracts thereof, safflower and extracts thereof, saffron and extracts thereof, and lemon balm and extracts thereof.

(12) An agent for women, wherein containing (A) a medium chain triacylglycerol, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides, and (C) one or two or more selected from the group consisting of γ-linolenic acid, dihome-γ-linolenic acid, calcium, magnesium, zinc, iron, vitamin B6, vitamin E, St. John's wort and extracts thereof, safflower and extracts thereof, saffron and extracts thereof, and lemon balm and extracts thereof.

(13) A fat and oil composition for body temperature elevation, which is comprised of a medium chain fatty acid and/or a glycerin fatty acid ester including the medium chain fatty acid as an active ingredient.

(14) A fat and oil composition for body temperature elevation, which is comprised of a medium chain fatty acid a glycerin fatty acid triester as an active ingredient.

(15) A food and drink for body temperature elevation containing a medium chain fatty acid and/or a glycerin fatty acid ester including the medium chain fatty acid.

(16) A food and drink for body temperature elevation containing a medium chain fatty acid glycerin fatty acid triester.

(17) A body temperature elevating agent raw material wherein containing a medium chain fatty acid and/or a glycerin fatty acid ester including the medium chain fatty acid.

(18) A body temperature elevating agent raw material wherein containing a medium chain triacylglycerol.

(19) A method for using a medium chain fatty acid and/or a glycerin fatty acid ester including the medium chain fatty acid as a body temperature elevating agent.

(20) A method for using a medium chain triacylglycerol as a body temperature elevating agent.

(21) Use for producing a body temperature elevating agent of a medium chain fatty acid and/or a glycerin fatty acid ester including the medium chain fatty acid.

BRIEF DESCRIPTION OF THE DRAWINGS

Fig. 1A shows oxidative phosphorylation and uncoupling by uncoupling protein (UCP) in a mitochondrial inner membrane.

Fig. 1B shows actions of uncoupling protein (UCP) and other functions in an adipose cell for a burning process of fats.

PREFERRED EMBODIMENTS

"Description of terms and description of body temperature elevating function by burning fats"

The following description refers to "Saito, Bessatsu Igaku no Ayumi, Adipose cells: Basic research and clinical application, 75-78, 1999". Burning of free fatty acids is primarily performed in a mitochondria. UCP is a protein which uncouples oxidative phosphorylation in the mitochondria. That is, typically, an electron transfer system and an ATP synthesis are closely coupled through an electrochemical gradient of protons via an inner membrane in the mitochondria. UCP is a special channel which dissolves this concentration gradient with a short-circuit. When this is activated, chemical energy of an oxidation substrate is not utilized for the ATP synthesis and is converted to heat. That is, the burning of free fatty acids is facilitated by the expression of UCPs. A burning process of neutral fats in an adipose cell is shown in Figs. 1A and 1B.

Here, in UCPs, UCP-1 to UCP-3 have been identified. UCP-1 is present in brown adipose tissues. However, in many mammals including humans, UCP-1 is reduced along with growth, and it is difficult to visually identify it in adults. On the other hand, UCP-2 is ubiquitously present in the human body and UCP-3 is strongly expressed in skeletal muscles.

Thermogenesis by UCP-1 in brown adipose tissues is directly regulated by sympathetic nerves which abundantly distribute in these tissues. That is, norepinephrine (NE) secreted from nerve endings by facilitating activity of the sympathetic nerve activates adenylate cyclase through β-adrenaline receptors present at a cell membrane to make fatty acids free by a series of reactions such as elevation of cAMP concentration, activation of protein kinases and degradation of stored neutral fats. The above free fatty acids are burnt through UCP-1 in the mitochondria. Therefore, the increase of an expressed amount of UCP facilitates the burning of fats in vivo. Hereinafter, the invention is described in detail.

[Aspects of use of the invention]

In the invention, a glycerin fatty acid ester including a medium chain fatty acid indicates a glycerin fatty acid ester which is comprised of fatty acids selected from carbon numbers 6 to 24 where one or more of either constituent fatty acids $R^1$, $R^2$ and $R^3$ shown in the following general formula (I) includes the aforementioned medium chain fatty acid. Particularly, the glycerin fatty acid esters are composed of a glycerin fatty acid triester having all constituent fatty acids $R^1$, $R^2$ and $R^3$ shown in the following general formula (I), a glycerin fatty acid diester where any one of constituent fatty acids $R^1$, $R^2$ and $R^3$ is hydrogen, and a glycerin fatty acid monoester where either only one is the constituent fatty acid. Furthermore, depending on binding sites, the glycerin fatty acid diesters having the constituent fatty acids at $R^1$ and $R^3$, the glycerin fatty acid diesters having the constituent fatty acids at $R^1$ and $R^2$, the glycerin fatty acid monoesters having the constituent fatty acid at only $R^1$ or $R^3$, and the glycerin fatty acid monoesters having the constituent fatty acid only at $R^2$ are included.

General formula (I)

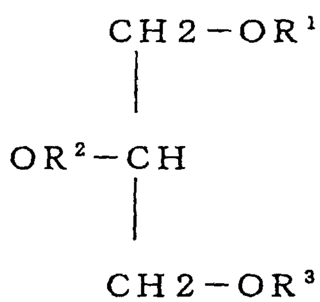

Here, in the glycerin fatty acid triesters, as forms comprising the medium chain fatty acids, there are those in which all constituent fatty acids $R^1$, $R^2$ and $R^3$ shown in the following general formula (I) are composed of the aforementioned medium chain fatty acids, those which any one of $R^1$, $R^2$ and $R^3$ is composed of the fatty acid selected from the carbon numbers 6 to 24, those which only $R^2$ is composed of the fatty acid selected from the carbon numbers 6 to 24, those which $R^1$ and $R^2$ or $R^3$ and $R^2$ are composed of the fatty acids selected from the carbon numbers 6 to 24, and those in which $R^1$ and $R^3$ are composed of the fatty acids selected from the carbon numbers 6 to 24. Those in which all constituent fatty acids $R^1$, $R^2$ and $R^3$ have the form of the aforementioned medium chain fatty acid are referred to a medium chain triacylglycerol, and this will be described later.

Here, when the medium chain fatty acid residues at positions 1 and 3 is 40% or more, preferably 50% or more, more preferably 60% or more, especially preferably 70% or more, and most preferably 80% or more by mass with respect to the total amount of the glycerin fatty acid ester comprising the above medium chain fatty acid, it is preferable, for example, because degradation and absorption are good in the intestine and the like when orally digested.

In these cases, it is possible to obtain the glycerin fatty acid ester including the medium chain fatty acid by performing an ester exchange treatment especially using a medium chain fatty acid glycerin fatty acid triester per se or a fat and oil highly containing the medium chain fatty acid glycerin fatty acid triester as a raw material.

As the glycerin fatty acid ester including the medium chain fatty acid, as especially preferable forms, it is preferable to comprise medium chain triacylglycerol, and if the medium chain fatty acid is the same in amount, it is preferable to be MLCT, and further it is preferable to be a structure where types and locations of the fatty acids are designed.

Contents can be defined by referring to the content of the medium chain fatty acid depending on strength of required functions. For example, the medium chain triacylglycerol may be 3 to 30%, preferably 3 to 23%, preferably 4 to 20%, more preferably 5 to 17%, especially preferably 6 to 15%, most preferably 7 to 14%, and most especially preferably 8 to 13% by mass. Furthermore, in the case of MLCT, the content may be 1 to 80%, preferably 2 to 70%, more preferably 3 to 60%, especially preferably 5 to 50%, most preferably 7 to 20%, and most especially preferably 8 to 14% by mass. Moreover, it is preferable to be a structure (structural fat and oil) where types of fatty acids and locations bound to glycerin are designed under consideration for imparting digestion and absorption functionality such as absorbability, easy/difficult degradability for lipase and dispersibility, and other biological chemical functions. In this case, as the structure, the content may be 1 to 80%, preferably 2 to 70%, more preferably 3 to 60%, especially preferably 5 to 50%, most preferably 7 to 20%, and most especially preferably 8 to 14% by mass.

MLCT is a glycerin fatty acid ester including medium chain fatty acid and of which the number of the medium chain fatty acid is 1 or 2 and the others are long chain fatty acids. In the invention, absorption and functions in vivo are different depending on the number of the medium chain fatty acid and the binding locations, and thus it is preferable to appropriately regulate them for any purpose. In the case of enhancing the absorbability, MLCT which the medium chain fatty acid(s) is bound to one or more of positions 1 and 3, and especially both positions is preferable.

In the structural fat and oil, it is possible to design for types of fatty acids other than the medium chain fatty acid bound to glycerin and locations to which these are bound. Preferable structural fats and oils include, for example, those which the medium chain fatty acid is bound to one of or both positions 1 and 3 and a certain fatty acid is bound to the position 2.

As certain fatty acids, especially in terms of the existing amount in nature, straight chain fatty acids are preferable, further straight chain unsaturated fatty acids are preferable, and in these, especially, monovalent unsaturated fatty acids such as palmitoleic acid, oleic acids, vaccenic acid and erucic acid, n-6 type unsaturated fatty acids such as linoleic acid, γ-linolenic acid, bishomo-γ-linolenic acid and arachidonic acid, n-3 type unsaturated fatty acids such as α-linolenic acid, stearidonic acid,eicosatetraenoic acid,eicosapentaenoic acid, docosapentaenoic acid and docosahexaenoic acid, and conjugated fatty acids such as conjugated linoleic acid and α-eleostearic acid, and the like are preferable.

As examples of these structural fats and oils, it is possible to exemplify M-oleate-M, M-α-linolenate-M, M-γ-linolenate-M, M-eicosapentaenoate-M, M-docosapentaenoate-M, M-docosahexaenoate-M, M-conjugated linoleate-M, and the like.

These MLCT and structural fats and oils can be produced by an ester exchange reaction, and can be preferably produced using an enzyme having location specificity.

The medium chain triacylglycerol used in the invention is one generally referred to as MCT (Medium Chain Triacylglycerol), and is a monoacid group or a mixed acid group triacylglycerol composed of saturated fatty acids with 6 to 12 carbons, and preferably 8 to 10 carbons such as palm oil degradation fatty acids. For example, it is possible to use triacylglycerol of caprylic acid (C8)/capric acid (C10) = 60 to 75/25 to 40 (weight ratio). The triacylglycerol can be produced by esterifying the above medium chain fatty acid and glycerin by common procedures, however, use of a commercially available one is convenient.

Fat and oil compositions containing medium chain triacylglycerol include common edible fats and oils, for example, soybean oil, rapeseed oil, rapeseed oil with high oleic acid, corn oil, sesame oil, sesame salad oil, vinegarweed oil, flax seed oil, peanut oil, safflower oil, safflower oil with high oleic acid, sunflower seed oil, sunflower seed oil with high oleic acid, cotton seed oil, grape seed oil, macadamia nut oil, hazel nut oil, pumpkin seed oil, walnut oil, camellia oil, tea seed oil, perilla oil, borage oil, olive oil, rice bran oil, wheat germ oil, palm oil, palm kernel oil, fractionated coconut oil, cacao butter, beef tallow, lard, chicken fat, cream, fish oil, seal oil, algoid oil, fats and oils thereof made into low saturation by quality improvement, and hydrogenated fats and oils thereof, fractional fats and oils thereof, and the like.

Here, the medium chain fatty acid contained in the body temperature elevating agent of the invention is preferably a fatty acid with 6 to 12 carbons, and examples thereof include caproicacid, caprylicacid, capric acid and lauric acid. Also, saturated fatty acids with 8 to 10 carbons, especially caprylic acid and capric acid are preferable.

The glycerin fatty acid ester containing the medium chain fatty acid is obtained naturally and synthetically, and can be produced by an ester exchange treatment using fats and oils of fractionated coconut oil such as palm oil and palm kernel oil having the medium chain fatty acid with 6 to 12 carbons as a constituent fatty acid, however, the method is not limited thereto. Conditions for ester binding reaction is not especially limited, however, for example, it is also possible to obtain by reacting under pressure with no catalyst and no solvent. Certainly, it is possible to obtain the glycerin fatty acid ester containing the medium chain fatty acid of the invention by a reaction using a catalyst and a solvent. Also, it is possible to obtain the glycerin fatty acid ester itself containing the medium chain fatty acid from oil stuff seeds of genetically modified plants or produce the glycerin fatty acid ester containing the medium chain fatty acid using the medium chain fatty acid obtained from oil stuff seeds of genetically modified plants as a raw material. Also, it is possible to obtain by an enzyme reaction using an enzyme. When considering flavor, taste, safety and/oral ingestion of the fats and oils, it is preferable to produce by the ester exchange treatment with an enzyme, however, the method is not limited thereto. Regardless of the composition of fatty acids at positions 1 and 3 and the composition of fatty acid at position 2 being biased or not biased, the more fatty acids there are at positions 1 and 3, the more preferable since degradation and the like tend to be more suitable, and thus it is preferable.

As mentioned above, a method for performing the ester exchange treatment using a lipid degrading enzyme (lipase) using fats and oils of fractionated coconut oil such as palm kernel oil having the medium chain fatty acid with 6 to 12 carbons as a constituent fatty acid is included. The enzymes include lipases derived from genus Alcaligenes, genus Candida, genus Rizopus, genus Mucor or genus Pseudomonas, phospholipase A derived from liver, and especially, lipases derived from genus Candida and genus Rizopus are preferable. Furthermore, for the enzymes, the type thereof can be appropriately selected depending on the conditions.

The method for performing the ester exchange reaction using the lipid degrading enzyme is not especially limited, and a specific method example is cited below. Temperature is adjusted to the range of 40 to 100°C which is the reaction temperature at which activity of the lipid degrading enzyme is sufficiently exerted. The lipid degrading enzyme was added thereto at a percentage of 0.005 to 10% by mass based on a raw material mixture, and the ester exchange reaction is performed in the range of 2 to 48 hours. It is desirable that this reaction is performed under normal pressure in nitrogen flow. Completion of the reaction is identified by measuring a triacylglycerol composition of a reaction product by gas chromatography. The reaction product is washed with water, dried, and subsequently decolorized and deodorized by common procedures. When the medium chain fatty acid is used, it is suitable that free fatty acids after stopping the reaction have been eliminated by a thin membrane type evaporator.

The body temperature elevating agent of the invention can also be produced by combining the purified medium chain fatty acid and/or the glycerin fatty acid ester including the medium chain fatty acid, or by combining/mixing fats and oils containing these.

A content of medium chain fatty acid and/or the glycerin fatty acid ester including the medium chain fatty acid with respect to the whole body temperature elevating agent of the invention is not especially limited so long as it is contained to an extent that it has a body temperature elevating action. Here, containing as an active ingredient is containing at an extent to exert its facilitation of UCP expression, however, its content is not especially limited, and may be appropriately regulated depending on the frequency of ingestion, the ingested amount, and the purpose of use. The content is not especially limited, however, for example, is 1% or more, preferably 5% or more, more preferably 5 to 99.9%, still preferably 10 to 99.9%, and especially preferably 14 to 99.9% by mass.

Using the medium chain fatty acid and/or the glycerin fatty acid ester including the medium chain fatty acid, that is, due to the presence of the medium chain fatty acid, various functions occur. Here, characteristics of its effects are also different due to its content and structure of the glycerin fatty acid ester.

For the content, being in the range in which the functions are obtained is required, preferably the content is in the above range, and the lower potential harmful effects on human bodies are more preferable, and further the lower content is more preferable commercially and industrially.

Furthermore, strength of its effects, temporal features (quick-acting, long-acting), and occurrences of other factors are different depending on the difference in absorption pathways due to the structures and the like. Taking advantage of this difference in structures, it is possible to perform designs of functional strength and temporal features, and designs of safety. For example, it is possible to exemplify fatty acid ester where at least one or more of the medium

chain fatty acids are bound to glycerin and especially glycerin fatty acid ester (hereinafter, referred to as "structural body" or "structural fat and oil") designed by specifying positions to which the medium chain fatty acid is bound and the types of other fatty acids. The contents of the medium chain fatty acid, an MLCT and a structural fat and oil in medicaments containing the MLCT and the structural body (structural fat and oil) can be referred to as in the above.

Here, as mentioned above, it is required that the content of the medium chain fatty acid is in the range in which the functions are obtained, preferably the content is in the above range, and the lower potential harmful effects on human bodies are more preferable, and further the lower content is more preferable commercially and industrially.

Considering the above point of view, for example, when intending to ingest for a long time or improve the constitution, the percentage of the medium chain fatty acid occupying the whole fatty acids may be 3 to 23%, preferably 4 to 20%, more preferably 5 to 17%, especially preferably 6 to 15%, most preferably 7 to 14%, and most especially preferably 8 to 13% by mass.

Animal experiments were performed regarding the relationship of the effects of the medium chain fatty acid and/or the glycerin fatty acid ester including the medium chain fatty acid of the invention with the content thereof when the invention is used as an agent, and the following is obtained. That is, expression amounts of UCP-2 of those given experimental diets of 100% by mass of long chain fatty acid, 100% by mass of the medium chain fatty acid and 25% by mass of the medium chain fatty acid were compared. As a result, compared to 100% by mass of the long chain fatty acid, the expression amounts of UCP-2 of those given the other two type experimental diets were remarkable. However, there was no significant difference in the expression amounts of UCP-2 between those given the experimental diet of 100% by mass of the medium chain fatty acid and those given the experimental diet of 25% by mass of the medium chain fatty acid. That is, the glycerin fatty acid ester including the medium chain fatty acid which is a major ingredient of the invention may be contained to the extent capable of exerting its effects.

It is preferable that the body temperature elevating agent is comprised of the medium chain fatty acid and/or the glycerin fatty acid ester including the medium chain fatty acid as a primary ingredient. Here, it is possible as a matter of course to combine ingredients composing formulations mentioned below as the other ingredients.

Here the body temperature elevating agent of the invention can be safely administered orally and parenterally to humans and animals as pharmaceuticals and quasi-drugs. Parenteral administration includes, for example, intravenous injection, arterial injection, intramuscular injection, subcutaneous injection, intradermal injection, intraperitoneal injection, intraspinal injection, extradural injection, percutaneous administration, intrapulmonary administration, intranasal administration, enteral administration, buccal administration, transmucosal administration, and the like, and dosage forms thereof include, for example, an injection agent, suppository (anal suppository, urethral suppository, vaginal suppository, etc.), liquid agent for external use (filling agent, collutorium, mouth washes, wet dressing, inhalation, air spray, aerosol agent, enema, endermic liniment, bed bath agent, decontaminating chemical, nose drop, ear drop, etc.), patch, percutaneous absorbable tape, cutaneous external medicine, ointment (pasta agent, linimentum agent, lotion agent, etc.), and the like. Also, orally administered formulations include, for example, tablets for internal use (uncoated tablets, sugar-coated tablets, coating tablets, enteric coated tablets, chewable tablets, etc.), intraoral tablets (buccal tablets, sublingual tablets, trochisci tablets, adherent agent, etc.), powdered medicine, capsule agents (hard capsules, soft capsules, etc.), granular agents (coating agents, pills, trochisci agents, liquid agents or pharmaceutically acceptable sustained release formulations thereof), and the like. Liquid agents for oral administration include, but are not limited to, for example, a water agent for internal use, shaking medical mixture, suspension agent, emulsion, syrup agent, dry syrup agent, elixir agent, immersion agent, decoction, limonade agent, and the like.

These formulations are administered as pharmaceutical compositions along with a pharmaceutically acceptable base, carrier, excipient, binder, disintegrant, lubricant, coloring agent and the like as formulations according to methods for pharmaceutical production publicly known.

The carriers and the excipients used for these formulations include, for example, sugars (lactose, saccharose, glucose, etc.), starch (maize, potato, wheat) mannitol, calcium carbonate, calcium phosphate, calcium sulfate, crystalline cellulose, microcrystalline cellulose, licorice powder, gentiana powder, and the like.

The binders used for these formulations include, for example, starch, tragacanth, gum, gelatin, syrup, polyvinyl alcohol, polyvinyl ether, polyvinyl pyrrolidone, hydroxypropylcellulose, methylcellulose, ethylcellulose, carboxymethylcellulose and the like.

The disintegrants used for these formulations include, for example, starch, agar, gelatin powder, sodium carboxymethylcellulose, calcium carboxymethylcellulose, crystalline cellulose, calcium carbonate, sodium hydrogen carbonate, sodium alginate and the like.

The lubricants used for these formulations include, for example, magnesium stearate, talc, hydrogenated plant oil, macrogol, and the like.

As the coloring agents used for these formulations, it is possible to use those accepted for use as pharmaceuticals.

Also when preparing the injection agent, each injection agent is made by a standard method by adding a pH adjuster, buffer, stabilizing agent, solubilizing agent and the like if necessary.

When preparing the tablets and the granular agents, they may be coated with saccharose, gelatin, hydroxypropylcellulose, purified shellac, gelatin, glycerin, sorbitol, ethylcellulose, hydroxypropylcellulose, hydroxypropylmethylcellulose, polyvinyl pyrrolidone, phthalate cellulose acetate, hydroxypropylmethylcellulose phthalate, methyl methacrylate, methacrylate polymer and the like if necessary, or may be coated with two or more layers, and furthermaybe capsules of substances such as ethylcellulose and gelatin.

Forms of external medicines include solid, semisolid or liquid formulations for transmucosal administration such as percutaneous, intraoral or nasal administration.

Liquid formulations include, for example, pharmaceutically acceptable emulsion agents such as emulsion or lotion, tincture agents for external use, liquid agents for transmucosal administration and the like. This formulation comprises, for example, ethanol, an oil content, emulsifying agent, and the like as diluents typically used.

Semisolid formulations include, for example, ointments such as oily ointments and hydrophilic ointments. This formulation comprises, for example, water, petrolatum, polyethyleneglycol, an oil content, surfactant and the like as bases and carriers typically used.

Semisolid or solid formulations include, for example, patches and the like for percutaneous administration or transmucosal administration (intraoral, nasal) such as emplastrum (rubber plaster, plaster, etc.), film agent, tape agent or cataplasm. This formulation comprises, for example, natural rubbers, rubber type high molecules such as synthetic rubbers such as butadiene gum, SBR and SIS, sludging agents such as gelatin, kaolin and zinc oxide, hydrophilic high molecules such as sodium carboxymethylcellulose and sodium polyacrylate, tackifiers such as acrylic resin and liquid paraffin, water, other oil contents, and surfactants as bases and carriers typically used.

Auxiliary agents such as a stabilizing agent, dissolving aid and percutaneous absorption accelerator, or additives such as a perfume and preservative may further be used for these formulations.

The body temperature elevating agent of the invention is wherein containing the medium chain fatty acid and/or the glycerin fatty acid ester including the medium chain fatty acid as mentioned above, its intended use is arbitrary, and can be used as the body temperature elevating agent or the raw material thereof in broad fields such as pharmaceuticals, quasi-drugs, medical foods and health foods. A combined amount to the body temperature elevating agent of the invention at that time is not completely defined because it is different depending on conditions such as intended use; dosage form; type, age, gender, body weight, degree of symptom and health state of a subject, however, it is contained to the extent that the body temperature elevating function is exerted.

The body temperature elevating agent of the invention has the body temperature elevating effect as mentioned above. That is, it is possible to enjoy the body temperature elevating effect by directly or indirectly orally or parenterally administering the body temperature elevating agent of the invention. Furthermore it is possible to obtain a more suitable effect by continuously ingesting. The combined amount of the body temperature elevating agent of the invention is not completely defined, and could be appropriately determined depending on strength of the effect to be required by considering the type, a use period and an amount of the constituent fatty acid; age, gender and body weight of the subject; whether directly orally ingested; whether combined as a raw material; and the like.

It is known that an uncoupling function is involved in diet inducedbodythermogenesis (Saito, Bessatsu, IgakunoAyumi, Adipose cells: Basic research and clinical application, pages 75-78, 1999). The uncoupling is referred to as inhibition of coupling of energy obtained by electron transport to ATP synthetic reaction in oxidative phosphorylation in vivo, and is one of the important biofunctions in terms of exchanging chemical energy coupled to the ATP synthetic reaction to thermal energy. UCP plays a central role in such an uncoupling function as an uncoupling substance present in the living body. The body temperature elevating agent of the invention has the body temperature elevating function through a facilitating effect of uncoupling protein expression as mentioned above.

Here, the uncoupling protein (UCP) is a special protein present in brown adipose tissues of rodents and hibernants, and it is known as a thermogenic molecule which uncouples oxidative phosphorylation in mitochondria to scatter oxidation energy as heat. Recently, it has been demonstrated that UCP is expressed inmany tis sues and cells including s keletal muscles in humans. Homologues have been identified in UCP and are different in distribution depending on tissues and cells (e.g., see Saito M., Ohashi A., Nippon Yakurigaku Zasshi, 118:327-333, 2001). The facilitation of the uncoupling protein expression is referred to as a state where a gene expression amount of the uncoupling protein is enhanced compared to normal, and can be assessed by molecular biological techniques. The molecular biological technique is referred to as a quantitative RT-PCR method where total RNA is extracted from tissues/cells followed by being converted to DNA using reverse transcriptase, and PCR (polymerase chain reaction) is quantitatively performed.

The facilitating effect of the uncoupling protein expression of the body temperature elevating agent of the invention can be assessed by a test method using the quantitative RT-PCR method. According to this assessment method, it has been found to have the facilitating effect of the uncoupling protein expression which is 2.5 to 5 times in rat liver and 1.2 to 3 times in rat adipose tissue excelling in rats for which the body temperature elevating agent has not been administered.

The body temperature elevating agent of the invention has preventive and/or improving effects of lifestyle

related diseases such as obesity and a low accumulation effect where body fat becomes difficult to be accumulated by smoothly consuming excessive energy and performing thermogenesis.

Since the invention article shows the body temperature elevating action as mentioned above, the direct effects such as obesity prevention effect, obesity improving effect and low accumulation effect of body fat as well as preventive and/or improving effects on lifestyle related diseases such as diabetes, arteriosclerosis, hyperlipemia and hypertension, or a losing weight effect through these effects are anticipated.

The body temperature elevating agent of the invention can combine one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides as those which additively and/or synergistically enhance the effects. This can improve the body temperature elevating function, is further effective for poor blood circulation, and further has suitable effects on one symptom of PMS.

Furthermore, it is possible to combine one or two or more selected from the group consisting of γ-linolenic acid, dihome-γ-linolenic acid, mineral salts such as calcium, magnesium, zinc and iron, vitamins such as vitamin B6 and vitamin dried herbs such as vitamin E, St. John's wort, safflower, saffron and lemon balm, and extracts thereof. The combination of these is a more preferable aspect which not only enhances the body temperature but also possesses the same functions together which these ingredients have. It is anticipated that the effects are improved by elevating the body temperature, and there are also the effects which provide fresh feeling and the like in the cases of orally ingesting. Additionally, since these are the ingredients which can address many symptoms of PMS in women, the invention is suitable as an agent for women containing these.

The preferable mode of the invention relates to the body temperature elevating agent which is comprised of the medium chain fatty acid glycerin fatty acid triester as an active ingredient. The medium chain triacylglycerol is as mentioned above.

With respect to the body temperature elevating agent of the invention, when using the medium chain triacylglycerol which is an especially preferable mode, the content thereof is not especially limited, however, for example, it is 0.5% or more, preferably 3% or more, more preferably 3 to 99.9%, still preferably 5 to 99.9%, especially preferably 8 to 99.9% and most preferably 15 to 99.9% by mass.

Also when containing as the primary ingredient, it is possible to exemplify 50 to 99.9%, preferably 70 to 99.9%, and more preferably 90 to 99.9% by mass.

Various functions occur by the presence of the medium chain fatty acid and/or the glycerin fatty acid ester including the medium chain fatty acid, i.e., the medium chain fatty acid. Here, characteristics of its effects are also different depending on its content and the structure of the glycerin fatty acid ester.

For the content, being in the range in which the functions are obtained is required, preferably the content is in the above range, and the lower potential harmful effects on human bodies are more preferable, and further the lower content is more preferable commercially and industrially.

Furthermore, strength of its effects, temporal features (quick-acting, long-acting), and occurrences of other factors are different depending on the difference of absorption pathways due to structures. Taking advantage of this difference in structures, it is possible to perform designs of functional strength and temporal features, and designs of safety. For example, it is possible to exemplify fatty acid ester (hereinafter referred to as "MLCT") where at least one or more of the medium chain fatty acids are bound to glycerin and especially glycerin fatty acid ester (hereinafter, referred to as "structural body" or "structural fat and oil") designed by specifying positions to which the medium chain fatty acid is bound and the types of other fatty acids. The contents of the medium chain fatty acid, an MLCT and a structural fat and oil in medicaments containing the MLCT and the structural body (structural fat and oil) can refer to the above.

Here, as mentioned above, it is required that the content of the medium chain fatty acid is in the range in which the functions are obtained, preferably the content is in the above range, and the lower potential harmful effects on human bodies are more preferable, and further the lower content is more preferable commercially and industrially.

Considering the above point of view, for example, when intending to ingest for a long time or improve the constitution, the percentage of the medium chain fatty acid occupying the whole fatty acids may be 3 to 23%, preferably 4 to 20%, more preferably 5 to 17%, especially preferably 6 to 15%, most preferably 7 to 14%, and most especially preferably 8 to 13% by mass.

The body temperature elevating agent of the invention is wherein containing the medium chain fatty acid and/or the glycerin fatty acid ester including the medium chain fatty acid as mentioned above, its intended use is arbitrary, and can be used as the body temperature elevating agent or the raw material thereof in broad fields such as pharmaceuticals, quasi-drugs, medical foods and health foods. A combined amount to the body temperature elevating agent of the invention at that time is not completely defined because it is different depending on conditions such as intended use; dosage form; type, age, gender, body weight, degree of symptom and health state of the subject, however, it is contained to the extent that the body temperature elevating function is exerted. Also the formulation having various

functions containing the body temperature elevating function of the invention contains as a matter of course to the extent that the functions of the effects on the prevention and/or the treatment of lifestyle related diseases such as diabetes are exerted. Also, the body temperature elevating agent of the invention is wherein containing the medium chain triacylglycerol as the preferable mode as mentioned above, its intended use is arbitrary, and can be used as the body temperature elevating agent or the raw material thereof in broad fields such as pharmaceuticals, quasi-drugs, medical foods and health foods. A combined amount to the body temperature elevating agent of the invention at that time is not completely defined because it is different depending on conditions such as intended use; dosage form; type, age, gender, body weight, degree of symptom and health state of a subject, however, it is contained to the extent that the body temperature elevating function is exerted.

The present invention relates to a fat and oil composition for body temperature elevation, which is comprised of medium chain fatty acid and/or glycerin fatty acid ester including the medium chain fatty acid as an active ingredient. Also preferably the invention relates to a fat and oil composition for body temperature elevation, which is comprised of medium chain fatty acid glycerin fatty acid triester as an active ingredient.

The medium chain fatty acid and/or the glycerin fatty acid ester including the medium chain fatty acid can be obtained from fats and oils, and is good in solubility in the fats and oils. Therefore, as one suitable mode of the body temperature elevating agent of the invention, foods and drinks for body temperature elevation shown below, the fat and oil composition containing the medium chain fatty acid and/or the glycerin fatty acid ester including the medium chain fatty acid is included. Also, when producing the glycerin fatty acid ester including the medium chain fatty acid, it is suitable because the fat and oil composition has been produced.

The content of the medium chain fatty acid and/or the glycerin fatty acid ester including the medium chain fatty acid, or the medium chain fatty acid glycerin fatty acid triester in the fat and oil composition is the content of an agent when utilized as the agent, and the content of a food and drink when used as the food and drink. Here, when used as the fat and oil for cooking, the content is different depending on a use amount for the food and drink, however, for example, it is possible to exemplify the contents of 10 to 90%, preferably 20 to 80% or more, and more preferably 30 to 70% or more by mass. When used for cooking, it is not preferable to make excessively high concentrations in terms of cooking suitability such as oil splatter and soot, and thus coordination with the use amount is required.

Various functions occur by the presence of the medium chain fatty acid and/or glycerin fatty acid ester including the medium chain fatty acid, i.e., the medium chain fatty acid. Here, characteristics of their effects are also different depending on its content and the structure of the glycerin fatty acid ester.

For the content, being in the range in which the functions are obtained is required, preferably the content is in the above range, and the lower potential harmful effects on human bodies are more preferable, and further the lower content is more preferable commercially and industrially.

Furthermore, strength of its effects, temporal features (quick-acting, long-acting), and occurrences of other factors are different depending on the differences in absorption pathways due to the structures. Taking advantage of this difference in structures, it is possible to perform designs of functional strength and temporal features, and designs of safety. For example, it is possible to exemplify fatty acid ester (hereinafter referred to as "MLCT") where at least one or more of the medium chain fatty acids are bound to glycerin and especially glycerin fatty acid ester (hereinafter, referred to as "structural body" or "structural fat and oil") designed by specifying positions to which the medium chain fatty acid is bound and the types of other fatty acids. The contents of the medium chain fatty acid, MLCT and the structural fat and oil in medicaments containing the MLCT and the structural body (structural fat and oil) can refer to the above.

Here, as mentioned above, it is required that the content of the medium chain fatty acid is in the range in which the functions are obtained, preferably the content is in the above range, and the lower potential harmful effects on human bodies are more preferable, and further the lower content is more preferable commercially and industrially.

Considering the above point of view, for example, when intending to ingest for a long time or improve the constitution, the percentage of the medium chain fatty acid occupying the whole fatty acids may be 3 to 23%, preferably 4 to 20%, more preferably 5 to 17%, especially preferably 6 to 15%, most preferably 7 to 14%, and most especially preferably 8 to 13% by mass.

Forms of the fat and oil composition may be any of liquid, solid and semisolid, and are not especially limited. The fat and oil composition of the invention is one mode of the above body temperature elevating agent, and one mode of the following food and drink for body temperature elevation, and the mode as the raw material combined thereto. Also, it can be used for cooking, as a result of use therefore, it is contained in the food and drink, and thus it indirectly influences the food and drink.

The fat and oil composition of the invention can be obtained by the ester exchange treatment using the fat and oil which contains the medium chain fatty acid glycerin fatty acid triester as the raw material as mentioned above. Also, it is possible to obtain the fat and oil composition of the invention by extracting from plants, e.g., soybean, rapeseed, corn, palm tree, palm, olive, flax seed, sunflower, safflower, camellia, cotton seed, Crea and the like, selectively bred to produce the fat and oil composition of the invention using gene recombination technology.

The fat and oil composition of the invention obtained as the above can be used as the fat and oil composition for cooking as it is or by combining additives typically used for the fat and oil composition for cooking.

Such additives include polyglycerin fatty acid ester, sucrose fatty acid ester, sorbitan fatty acid ester and the like for the purpose of storage stability improvements, oxidation stability improvements, heat stability improvements, crystallization inhibition at low temperature treatment, and the like. Also it is as a matter of course that the additives and the like capable of being added in the above temperature elevating agent can be combined.

The fat and oil composition of the invention possesses flavor equivalent to or more than usual edible oils commonly marketed such as rapeseed oil, corn oil, safflower oil and soybean oil, as a matter of course it is able to be used for cooking such as stir-fries, fried foods and marinating, and is capable of being used also for dressings, mayonnaise, margarine, confectioneries, cakes, drinks, and the like. Flavor properties are different depending on the kinds of cooking articles, and it is possible to make fresh cuisine by utilizing the taste of the materials. Splattering degree of oil during by cooking equivalent to or less than that of usual edible oils.

The invention relates to a food and drink for body temperature elevation containing a medium chain fatty acid and/or glycerin fatty acid ester including the medium chain fatty acid, and preferably relates to a food and drink for body temperature elevation containing a medium chain fatty acid glycerin fatty acid triester. The food and drink containing the medium chain fatty acid and/or the glycerin fatty acid ester including the medium chain fatty acid, preferably the medium chain fatty acid glycerin fatty acid triester is suitably orally ingested by drinking and eating, shows body temperature elevating action, and thus is a preferable mode.

The preferable mode which is comprised of the medium chain fatty acid and/or glycerin fatty acid ester including the medium chain fatty acid, preferably the medium chain triacylglycerol fatty acid express the body temperature elevating effect includes oral ingestion, and especially the mode of foods and drinks is preferable to ingest routinely and continuously. As the food and drink for the body temperature elevation, the content can be regulated by a frequency, an amount of ingestion and the like as mentioned above, and is not especially limited. In the case of the medium chain fatty acid glycerin fatty acid triester, it is possible to exemplify, for example, from 0.2 to 50%, preferably 0.5 to 40%, more preferably 1 to 20%, and especially preferably 2 to 10% by weight. Also, in the case of the medium chain fatty acid glycerin fatty acid triester, it is possible to exemplify, for example, from 0.1 to 30%, preferably 0.2 to 20%, more preferably 0. 5 to 10%, and especially preferably 1 to 5% by weight.

Here, the content in the above body temperature elevating agent and the food and drink for the body temperature elevation can also, be defined by a total amount of the medium chain fatty acid, or the medium chain fatty acid and medium chain fatty acid residues, and the suitable content can be converted from the content of the medium fatty acid chain glycerin fatty acid triester.

Various functions occur by the presence of the medium chain fatty acid and/or glycerin fatty acid ester including the medium chain fatty acid, i.e., the medium chain fatty acid. Here, characteristics of their effects are also different depending on its content and the structure of the glycerin fatty acid ester.

For the content, being in the range in which the functions are obtained is required, preferably the content is in the above range, and the lower potential harmful effects on human bodies are more preferable, and further the lower content is more preferable commercially and industrially.

Furthermore, strength of its effects, temporal features (quick-acting, long-acting) , and occurrences of other factors are different depending on the difference in absorption pathways due to the structures. Taking advantage of this difference in structures, it is possible to perform designs of functional strength and temporal features, and designs of safety. For example, it is possible to exemplify fatty acid ester (hereinafter referred to as "MLCT") where at least one or more of the medium chain fatty acids are bound to glycerin and especially glycerin fatty acid ester (hereinafter, referred to as "structural body" or "structural fat and oil") designed by specifying positions to which the medium chain fatty acid is bound and the types of other fatty acids. The contents of the medium chain fatty acid, an MLCT and a structural fat and oil in medicaments containing the MLCT and the structural body (structural fat and oil) can refer to the above.

Here, as mentioned above, it is required that the content of the medium chain fatty acid is in the range in which the functions are obtained, preferably the content is in the above range, and the lower potential harmful effects on human bodies are more preferable, and further the lower content is more preferable commercially and industrially.

Forms of the foods and drinks for the body temperature elevation of the invention are not especially limited, and include, for example, forms where the foods and drinks are drinks, nutrition supplement drinks, confectionery, processed foods, fats and oils, milk products, retort foods, foods for microwave, frozen foods, seasonings, health supplements, and the like. Shapes and characters are also not especially limited, may be any of solid, semisolid, gel, liquid, powder and the like, and may be any of tablets, capsules, liquid agents, granules and the like.

Other physiologically active ingredients and the like can be combined in the foods and drinks for body temperature elevation for the purpose of enhancing various functions. They include anti-oxidant ingredients; oily ingredients; various vitamins, minerals, amino acids and the like for nutritional enrichment. The anti-oxidant ingredients are not especially limited, and include tocopherols and derivatives thereof, tocotrienols and derivatives thereof, lignans

such as sesamin, episesamin, sesaminol, sesamolin and sesamol and glucosides thereof, carotenoids such as β-carotene and derivatives thereof, tannins such as gallic acid and ellagic acid and derivatives thereof, flavonoids such as flavone, catechin, quercetin and leucoanthocyanidin, quinones such as ubiquinone and vitamin K, ferulic acid derivatives such as orizanol, olive extracts and the like. Types of various vitamins, minerals and amino acids for nutritional enrichment are not especially limited, however, those defined in Japanese Standard of Food Additives are desirable.

Concerning the foods and drinks for body temperature elevation of the invention, specific examples are listed below, however, the invention is not limited thereto. The foods and drinks for body temperature elevation of the invention are not especially limited in forms thereof, and include Japanese confectioneries such as sliced and dried rice cake, Japanese cracker, waxy rice fixed with starch syrup, bun with bean-jam filling and glutinous rice jelly, various Western confectioneries such as cookie, biscuit, cracker, pie, castilla, doughnut, pudding, sponge cake, waffle, butter cream, custard cream, cream puff, chocolate, chocolate cake, caramel, candy, chewing gum, jelly, hot cake, bread and sweet bread, snacks such as potato crisp, water ices such as ice cream, ice candy and sherbet, soft drinks such as lactic acid beverages, lactic acid bacteria beverages, rich milk beverages, fruit juices, fruit pulp beverages, functional drinks and carbonated beverages, favorite foods and drinks such as green tea, tea, coffee and cocoa, milk products such as fermented milk, processed milk and cheese, soybean processed foods such as soybean milk and soybean curd, pastes such as jam, fruit pickled with syrup, flower paste, peanut paste and fruit paste, pickles, grain products such as noodles and pasta, animal meat products such as ham, sausage, bacon, dried sausage, jerked beef and hamburger, seafood products such as fish ham, fish sausage, steamed fish paste, fish cake with a hole, fish minced and steamed, dry foods of fish and shell fish, boiled and dried flesh of bonito, mackerel, and scad, salted fermented seafood products of sea urchin and squid, dried squid and fish dried with Japanese sweet rice wine for cooking, smoke-dried salmon and the like, laver, small fish, shellfish, wild vegetable, oriental black mushroom, kelp and the like boiled down in soy sauce, retort foods of curry and stew, various seasonings such as soybean paste, soy sauce, sauce, ketchup, bouillon, mop sauce for grilled beef, curry roux, instant stew, instant soup and seasoning soy sauce mix, rices, fat and oil processing goods such as fats and oils, margarine, shortening, mayonnaise and dressing, various foods for microwave and frozen foods containing fats and oils, and the like. In terms of continuous ingestion, it can be said that rice, various seasonings, and fat and oil processing goods such as fats and oils, margarine, shortening, mayonnaise and dressing are preferable. Also the shapes and characters are not especially limited, and may be any of solid, semisolid, gel, liquid, powder and the like. When used as a health supplement foods and drinks, the shapes may be any of tablets, capsules filled and processed in soft capsules or hard capsules, liquid agents, granules and the like.

The especially preferable mode is food and drink in the form of the fat and oil composition, and food and drink cooked or combined using the fat and oil composition. As mentioned above, since the medium chain triacylglycerol and the like are fats and oils and the fats and oil composition can be obtained in the process of the production of the medium chain triacylglycerol, the fat and oil composition is excellent in usability, stability and productivity.

Also, as mentioned above, it is possible to combine the above body temperature elevating agent and the fat and oil composition. Also, it is possible to obtain by cooking using the fat and oil composition. Also, especially, when lipid contents included in the food and drink are composed of the body temperature elevating agent or the fat and oil composition for liver, or the like, its effects are suitable.

The invention relates to a body temperature elevating agent raw material wherein containing medium chain fatty acid and/or glycerin fatty acid ester including the medium chain fatty acid, and especially relates to a body temperature elevating agent raw material wherein containing medium chain triacylglycerol.

The body temperature elevating agent and the like of the invention are wherein containing the medium chain fatty acid and/or the glycerin fatty acid ester including the medium chain fatty acid, especially wherein containing the medium chain triacylglycerol, thereby having the body temperature elevating effect. Therefore, those containing the medium chain fatty acid and/or the glycerin fatty acid ester including the medium chain fatty acid, especially the medium chain triacylglycerol are suitable as the raw material of the body temperature elevating agent, and especially those containing at a high concentration are suitable as a raw material agent. The content is not especially limited, however, it is possible to exemplify, for example, from 50 to 99.9%, preferably 70 to 99.9%, and more preferably 90 to 99.9% by mass.

Various functions occur by the presence of the medium chain fatty acid and/or glycerin fatty acid ester including the medium chain fatty acid, i.e., the medium chain fatty acid. Here, characteristics of their effects are also different depending on its content and the structure of the glycerin fatty acid ester.

Furthermore, strength of its effects, temporal features (quick-acting, long-acting), and occurrences of other factors are different depending on the difference in absorption pathways due to the structures. Taking advantage of this difference in structures, it is possible to perform designs of functional strength and temporal features, and designs of safety. For example, it is possible to exemplify fatty acid ester (hereinafter referred to as "MLCT") where at least one or more of the medium chain fatty acids are bound to glycerin and especially glycerin fatty acid ester (hereinafter, referred to as "structural body" or "structural fat and oil") designed by specifying positions to which the medium chain fatty acid is bound and the types of other fatty acids.

The invention relates to the body temperature elevating agent which is comprised of the medium chain fatty acid and/or the glycerin fatty acid ester including the medium chain fatty acid as the active ingredient, and especially the body temperature elevating agent which is comprised of the medium chain fatty acid glycerin fatty acid triester as the active ingredient. Also, the invention relates to the fat and oil composition and the food and drink containing these. These medium chain fatty acids and/or glycerin fatty acid ester including the medium chain fatty acids are excellent in body temperature elevating effect. The medium chain fatty acid and/or the glycerin fatty acid ester including the medium chain fatty acid, especially the medium chain fatty acid glycerin fatty acid triester according to the invention can be suitably used for any of the agents or products obtained from natural plants and obtained artificially. Especially when used as the body temperature elevating agent raw material, the higher purity is more preferable.

In the invention, it has been found that the medium chain fatty acid and/or the glycerin fatty acid ester including the medium chain fatty acid has the body temperature elevating function. It is possible to make the body temperature elevating agent more preferable by further enhancing and complementing this function.

Although already mentioned, it is possible to combine one or two or more selecting from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides as those which enhance its effects additively and synergistically with the body temperature elevating agent of the invention. This can improve the body temperature elevating function, is further effective for poor blood circulation, and additionally has a suitable effect on one symptom of PMS.

Especially, it is possible to design so as to regulate, for example, a body temperature elevating rate, a body temperature elevating duration, a body temperature elevating site and the like by combining those having a body temperature elevating property which is different from that of the medium chain fatty acid and/or the glycerin fatty acid ester including the medium chain fatty acid.

Furthermore, it is possible to combine one or two or more selected from the group consisting of γ-linolenic acid, dihome-γ-linolenic acid, minerals such as calcium, magnesium, zinc and iron, vitamins such as vitamin B6 and vitamin E, dried herbs such as St. John's wort, safflower, saffron and lemon balm and extracts thereof. By combining these, an aspect that not only elevates the body temperature, but also has functions that these ingredients have together can be realized. It is anticipated to improve the effect by the body temperature elevation, and the effect has fresh feelingwhen orally ingested. Additionally, these ingredients can address many of the PMS symptoms in women, and thus it is suitable as an agent for women containing these.

Here, many women are suffering from poor blood circulation, and further poor blood circulation is one of large modes of PMS. Thus, it is possible to make a suitable formulation for women by further combining ingredients having effects on PMS other than poor blood circulation.

The invention contains the medium chain fatty acid and/or glycerin fatty acid ester including the medium chain fatty acid, preferably contains the medium chain triacylglycerol, and it is possible to obtain an agent for women by further combining ingredients having improving effects on PMS. As the ingredients having the improving effects on PMS, it is possible to exemplify the following, however, the ingredients are not limited thereto.

As mentioned above, it is possible to combine one or two or more selected from the group consisting of γ-linolenic acid, dihome-γ-linolenic acid, minerals such as calcium, magnesium, zinc and iron, vitamins such as vitamin B6 and vitamin E, dried herbs such as St. John's wort, safflower, saffron and lemon balm and extracts thereof. By combining these, an aspect that not only elevates the body temperature however, also has functions that these ingredients have together can be realized It is anticipated to improve the effect by the body temperature elevation, and the effect has fresh feelingwhen orally ingested. Additionally, these ingredients can address many of the PMS symptoms in women, and thus it is suitable as an agent for women containing these. In this case, it is possible to be made more suitable by combining the body temperature elevating agent exemplified in the above agent for poor blood circulation.

That is, as the preferable modes of the invention, it is possible to exemplify those containing the following ingredients (A), (B) and (C).

(A) Medium chain fatty acid and/or glycerin fatty acid ether comprising medium chain fatty acid;
(B) one or two or more selected from the group consisting of highly unsaturated fatty acid or fatty acid ester including this, conjugated linoleic acid or fatty acid ester including this, capsaicins, thiazolidine dione derivatives, genus Cirsium plants, black five grains, and pituitary adenylate cyclase activation peptides; and
(C) one or two or more selected from the group consisting of γ-linolenic acid, dihome-γ-linolenic acid, minerals such as calcium, magnesium, zinc and iron, vitamins such as vitamin B6 and vitamin E, dried herbs such as St. John's wort, safflower, saffron and lemon balm and extracts thereof.

PMS indicates physical symptom complex or mental symptom complex which occurs about one week before menstruation (menses) first reported by Frank in 1931, and is defined medically as "physical or mental symptoms which occur 3 to 10 days before the start of menstruation and attenuate or disappear along with the start of menstruation"

(Japan Society of Obstetrics and Gynecology, 1990). Also, this name PMS is also expressed as premenstrual tension. Furthermore, various expressions such as premenstrual experience have been used whereby women should be released from the negative image received from medical terms of premenstrual disorder syndrome or premenstrual tension, not being exploited by medical services due to symptoms and perform self-care positively.

The representative symptoms of PMS include lower abdominal pain, lower abdominal tension, lumber pain, mastodynia, mammary pain, stiff neck, headache, appetite increase, rough surface, acne, diarrhea, constipation, and the like. The mental symptoms include irritation, depression, being touchy, growing weary of doing things, talking abusively, getting the urge to organize, and the like.

It is inferred that numerous women before menopause have such PMS and symptoms and that a majority of the women before menopause have some symptoms though the severity of the symptoms vary. Moreover, serious patients sometimes bring about obstacles to daily life, and it is considered that the symptoms cause potential interpersonal aggravation at work sites, family and friendships, and even involvement in crime. Some scholars point out the relationship of work productivity of women with PMS under such circumstances where the advances into society by women have increased more and more.

With such circumstances, it seems to be a great social task to alleviate and to reduce and improve the QOL (quality of life) of women.

Examples

The following examples show that fat and oil compositions which are comprised of medium chain triacylglycerol as an active ingredient facilitates body temperature elevation, and more specifically illustrates the invention, however, the invention is not limited thereto.

[Example 1. Expression of UCP-2]

Fats accumulated in vivo are decomposed into free fatty acids, for example, by an action of hormone sensitive lipase. The free fatty acids are converted to thermal energy by burning in mitochondria. One which largely influences when the free fatty acids are burnt and converted to thermal energy is the UCP-2. The UCP-2 is expressed on a membrane which forms the mitochondria. The UCP-2 is a special channel which short circuits an inner reaction with an outer reaction of the membrane which forms the mitochondria. That is, the UCP-2 short circuits the other chemical reaction for the free fatty acid on the membrane of mitochondria to change it to heat. Therefore, when an expressed amount of the UCP-2 is abundant, burning of the free fatty acid is smoothly performed. In the example, experiments were performed using rats, and it was confirmed that oral ingestion of medium chain triacylglycerol which is a major ingredient of the invention was effective for the expression of UCP-2.

[Experimental conditions]

Wistar male rats aged 6 weeks were preliminarily kept for one week, subsequently divided into two groups (each 20 rats) of a group of soybean oil addition and a group of fat and oil composition addition containing 100% medium chain triacylglycerol, and given an experimental feed in which all sucrose of AIN93 standard feed is replaced with corn starch, and water ad libitum. Body weight and ingestion amounts were measured with time. The rats were kept for 6 weeks, killed after fasting for 18 hours, and afforded measurement of an expressed amount of UCP-2 in each site. The measured sites were liver and epididymis peripheral adipose tissue.

[Measurement of expressed amount of UCP-2]

Measurement of the expressed UCP-2 amount was carried out as follows. That is, tissue at each site of the rat to be measured was removed, homogenized by Isogen (supplied by Nippon Gene), and subsequently the total RNA in the tissue was extracted with chloroform and isopropyl alcohol to yield the total RNA. A concentration was measured by a spectrophotometer (Gene Quant: supplied by Pharmacia). A reverse transcriptase of M-MLV (supplied by Promega) was added, and reacted in the presence of an attached reaction buffer, 0.5 mM of dNTP (supplied by Invitrogen) and 25 μg/ml of oligo (dT) 15 primer (supplied by Promega) at 37°C for one hour to prepare cDNA. Using the prepared cDNA as a template, the expressed UCP-2 amount in the cDNA template derived from each site tissue was measured by a quantitative PCR apparatus (Light Cycler, supplied by Roche) by setting primers for rat UCP-2 and making the cDNA template of the corresponding tissue in the rat given solid feed (Lab MR-Stock, supplied by Nihon Nosan Kogyo K.K.) as a standard. For the expressed amount (%), relative values for the expressed amounts of β-actin were compared as shown in the mathematical formula 1.

<Mathematical formula 1>

Expressed amount of UCP-2 (%) = (Expressed amount of UCP-2 in rat/Expressed amounts of $\beta$-actin in rat) x 100

Facilitating effects of the UCP-2 expression were evaluated by the above method. The results are shown as the expressed amount (%) of UCP-2 in Table 1.

<Table 1>

| | Liver | | Epididymis Peripheral adipose | |
|---|---|---|---|---|
| | Average | SEM | Average | SEM |
| Soybean oil addition group | 79.3 | 20.3 | 222.5 | 19.4 |
| Fat and oil composition addition group | 214.1 | 60.4 | 265.8 | 71.3 |
| Significant difference | P<0.05 | | n.s. | |
| *Mann-Whitney Utest (Two-sided test) | | | | |

As a result of comparing the expressed amounts of UC-2 with those in the soybean oil addition group by the above method, the expressed amounts of UCP-2 were increased by about 2. 7 times in the liver and about 1.2 times in the epididymis Peripheral adipose in the fat and oil composition addition group compared to the soybean oil addition group. Thereby revealing that the fat and oil composition which is comprised of the medium chain triacylglycerol as an active ingredient has a facilitating effect of uncoupling protein expression.

[Example 2. Measurement of UCP-2 expression]

Expression of UCP-2 at each site of the rats was measured with the same purpose and techniques as those in Example 1. However, in this Example, fasting periods before the measurement were 6 and 18 hours. Also measured sites were liver, epididymis Peripheral adipose and subcutaneous adipose. The expressed amount of UCP was measured by the same techniques as those in Example 1. The results are shown in Table 2.

<Table 2>

| Fasting time period | | Liver | Epididymis Peripheral adipose | Subcutaneous adipose |
|---|---|---|---|---|
| 6h | LCT | 85.4±4.0 | 40.1±6.2 | 92.7±11.9 |
| | MCT | 78.0±6.0 | 47.0±11.5 | 244.0±44.7 |
| 18h | LCT | 41.8±0.4 | 107.8±22.4 | 199.8±36.3 |
| | MCT | 70.0±7.9 | 249.0±60.0 | 252.1±44.6 |
| LCT: Soybean oil addition group<br>MCT: Fat and oil composition addition group | | | | |

From the table, it is understood that the UCP-2 was significantly expressed in the subcutaneous adipose in a 6 hour fasting group and in the liver and the epididymis peripheral adipose in an 18 hour fasting group.

[Example 3. Comparison of expressed amount of UCP-2 in each site]

When adipose and the like are burnt in vivo, first the adipose is burnt and subsequently muscle is burnt. In this example, burning aspects of the adipose in each site in vivo when the medium chain triacylglycerol was administered were compared to those when not administered. A survey was performed by comparing changes with time of the expressed amounts of UCP-2 at each site.

[Survey method]

Wistar male rats aged 6 weeks were preliminarily kept for one week, subsequently divided into two groups

(each 20 rats) of a group of soybean oil addition and a group of fat and oil composition addition containing 100% medium chain triacylglycerol, and given an experimental feed in which all sucrose of AIN93 standard feed is replaced with corn starch, and water ad libitum. Body weight and ingestion amounts were measured with time. The rats were kept for 6 weeks, subsequently the rats afforded for the tests were starved for 6 hours, then killed, and afforded measurement of the expressed amount of UCP-2 in each site. The results are shown in Table 3.

<Table 3>

| Fasting time period | UCP-2/UCP-2' | | |
|---|---|---|---|
| | Liver | Epididymis Peripheral adipose | Subcutaneous adipose |
| 6h | 91.3 | 117.2 | 263.2 |

The results shown in the above table were obtained by comparing the expressed amounts of UCP-2 in the rats in the soybean oil addition group and in the rats in the medium chain triacylglycerol addition group according to the following mathematical formula 2.

<Mathematical formula 2>

Value in Table 4 = UCP-2/UCP2' x 100

UCP-2: UCP-2 in the rats in the fat and oil composition addition group
UCP-2': UCP-2 in the rats in the soybean oil addition group

From the above table, the following can be said. That is, the expression of UCP-2 in the subcutaneous adipose is obviously increased in the rats in the fat and oil composition addition group in comparison with the rats in the soybean oil adding group. That is, it is shown that in comparison with the expression of UCP-2 in the internal organ adipose, the increase of UCP-2 expression in the subcutaneous adipose is remarkable, indicating effects on uncoupling protein expression in the subcutaneous adipose.

[Example 4. Survey of effects with MCT contents]

In order to survey effects of contents at the ingestion of medium chain triacylglycerol (MCT), the following experiment was performed. Wister male rats aged 6 weeks were preliminarily kept for one week, and subsequently, divided into 3 groups of a soybean addition group, a fat and oil composition containing 100% medium chain triacylglycerol addition group and a fat and oil composition containing 25% medium chain triacylglycerol addition group. After fasting for 6 hours, 7 500 mg/kg per body weight of emulsified fat and oil was forcibly administered orally using a sonde. Rats in each group were killed in 60 min after the administration, and afforded measurement of the UCP-2 expression amount in the liver. The results are shown in Table 4.

<Table 4>

| Rat | Expressed amount of UCP-2 |
|---|---|
| LCT | 54.3±8.1 |
| MCT | 94.0±27.9 |
| MIX25 | 162.8±27.9 |

LCT: Soybean oil addition group
MCT: Fat and oil composition addition group
MIX 25: 25% MCT combination group

According to the table, the expressed amount of UCP-2 in the rats in the MIX 25 was increased. That is, it is shown that the expressed amount of UCP-2 in the liver immediately after the ingestion is maximum in the MIX 25 group, and followed by the MCT group. Numerical values of the expressed amounts of UCP-2 were obtained by the same technique as that in Example 1.

[Example 5. Comparison of dietary body thermogenic ability]

Wister male rats aged 8 weeks were kept with commercially available feed (Lab MR-Stock, supplied by Nihon Nosan Kogyo K.K.) for 2 days or more. After fasting for 18 hours, 5000 mg/kg body weight was forcibly administered orally using the sonde to the rats of two groups (each 8 rats) of a soybean addition group and a fat and oil composition containing 100% medium chain triacylglycerol addition group. Thereafter, the rats were placed in a metabolic measurement system for small animals (MK-5000R, supplied by Muromachi Kikai) where oxygen consumption and carbon dioxide production can be measured, and the oxygen consumption and the carbon dioxide production were measured for 6 hours after the administration. A consumed energy amount (energy consumption at rest) per hour was calculated from the obtained data, and dietary body thermogenic ability was compared.

The dietary body thermogenic ability was evaluated by the above method. The results are shown as dietary body thermogenic amounts in Table 5.

<Table 5>

| | Soybean oil addition group | Fat and oil composition addition group |
|---|---|---|
| Pre | 0.00 | 0.00 |
| 0 to one hour | 7.97 | 25.13** |
| 1 to 2 hours | 7.69 | 14.75* |
| 2 to 3 hours | 10.40 | 14.89 |
| 3 to 4 hours | 18.06 | 15.19 |
| 4 to 5 hours | 14.73 | 14.33 |
| 5 to 6 hours | 17.60 | 17.49 |
| Unit: cal/kg/min<br>Test: Mann-Whitney U-test (Two-sided test) | | |

*: P<0.05
**: P<0.01

<Dietary body thermogenic amount>

As a result of obtaining the consumed energy amounts by the above method, significant increase of the dietary body thermogenesis was observed during 0 to 2 hours in the fat and oil composition addition group, thereby revealing that the fat and oil composition which is comprised of the medium chain triacylglycerol as an active ingredient has a facilitating effect of uncoupling protein expression.

[Example 6. Warm up feeling test]

Emulsified fats and oils combined such that the content of the soybean oil or the fat and oil composition which is comprised of medium chain triacylglycerol as the active ingredient was 10% by mass were randomly given to six healthy adult women at fasting. Warm up feeling of the body after 60 min was determined by the following criteria and shown by an average score. The test was carried out twice, and rendered a crossover test of the soybean oil and the fat and oil composition.

Score 5: strong warm up feeling after the dose compared to before the dose;
Score 4: warm up feeling after the dose compared to before the dose;
Score 3: slight warm up feeling after the dose compared to before the dose;
Score 2: unconscious warm up feeling after the dose compared to before the dose; and
Score 1: equal feeling after the dose compared to before the dose.

The present test was carried out according to the Declaration of Helsinki. The warm up feeling was evaluated by the above method. The results are shown as the warm up feeling in Table 6.

<Table 6>

| | Soybean oil addition group | Fat and oil composition addition group |
|---|---|---|
| Subject A | 2 | 5 |
| Subject B | 3 | 4 |
| Subject C | 2 | 4 |
| Subject D | 3 | 5 |
| Subject E | 3 | 3 |
| Subject F | 2 | 4 |
| Average | 2.5 | 4.2 |

As a result of obtaining the warm up feeling by the above method, the warm up feeling of the body was more noticeable in the group taking the fat and oil composition. This has demonstrated that the fat and oil composition which is comprised of medium chain triacylglycerol as the active ingredient has a dietary body thermogenic effect.

[Example 7: Tablet composition]

By the combination ratio in Table 7, respective materials were thoroughly mixed, and this mixture was tableted to afford a tablet composition with one tablet of 300 mg.

<Table 7>

| | |
|---|---|
| Medium chain triacylglycerol | 60.0 mg |
| Maize starch | 14.5 mg |
| Crystalline cellulose | 125.0 mg |
| Calcium carboxymethylcellulose | 110.5 mg |

[Example 8: Capsule composition]

By the combination ratio in Table 8, one in which respective materials were thoroughly mixed was filled in capsules to afford a capsule composition.

<Table 8>

| | |
|---|---|
| Medium chain triacylglycerol | 150.0 mg |
| Lactose | 70.0 mg |
| Maize starch | 38.0 mg |
| Magnesium stearate | 2.0 mg |

[Example 9: Powder medicine]

By the combination ratio in Table 9, first medium chain triacylglycerol and lactose were thoroughly mixed, subsequently hydroxypropylcellulose was added and granulated. This was dried, subsequently grained, soft silicic acid anhydrate was added and thoroughly mixed to afford a powder medicine.

<Table 9>

| | |
|---|---|
| Medium chain triacylglycerol | 200.0 mg |
| Lactose | 791.0 mg |
| Hydroxypropylcellulose | 4.0 mg |
| Soft silicic acid anhydrate | 5.0 mg |

[Example 10: Gel ointment]

A gel ointment was prepared by the formulation shown in Table 10 and the following production method.

<Table 10>

| | (%) |
|---|---|
| (1) Carboxyvinyl polymer | 1.0 |
| (2) Triethanolamine | 1.0 |
| (3) 1,3-butyleneglycol | 10.0 |
| (4) Medium chain triacylglycerol | 30.0 |
| (5) Purified water | remaining amount |

(Production method)

A. Ingredients (1) and (3) to (5) are mixed and dissolved.
B. The ingredient (2) was added to A, mixed and made evenly to afford the gel ointment.

[Example 11: Emulsion agent]

An Emulsion was prepared by the formulation shown in Table 11 and the following production method.

<Table 11>

| | (%) |
|---|---|
| (1) Polyoxyethylene (10E.O.) sorbitan monostearate | 1.0 |
| (2) Polyoxyethylene (60E.O.) sorbit tetraoleate | 0.5 |
| (3) Glyceryl monostearate | 1.0 |
| (4) Stearic acid | 0.5 |
| (5) Behenyl alcohol | 0.5 |
| (6) Squalane | 8.0 |
| (7) Medium chain triacylglycerol | 20.0 |
| (8) Tocopherol acetate | 1.0 |
| (9) Preservative | 0.1 |
| (10) Carboxyvinyl polymer | 0.1 |
| (11) Sodium hydroxide | 0.05 |
| (12) Ethyl alcohol | 5.0 |
| (13) Purified water | to balance |
| (14) Perfume | appropriate |

(Production method)

A. Ingredients (9) to (13) are mixed with heating, and retained at 70°C.
B. Ingredients (1) to (6) are mixed with heating, and retained at 70°C.
C. A is added to B to emulsify evenly.
D. After cooling C, (7), (8) and (14) were added, and mixed evenly to afford the Emulsion.

[Example 12: Parenteral injection]

By the combination ratio shown in Table 12, first (1) and (2) were thoroughly mixed, and subsequently a total volume was made 1 ml by adding an appropriate amount of (3) to afford an injectable solution. This solution can be administered by diluting with an appropriate amount of saline.

<Table 12>

| | |
|---|---|
| (1) Medium chain triacylglycerol | 200.0 mg |

<Table 12> (continued)

| | |
|---|---|
| (2) Polyoxyethylene hardened castor oil | 200.0 mg |
| (3) Ethanol anhydrate | appropriate |

[Example 13: Soft drink]

Raw materials shown in Table 13 were mixed evenly to afford a health drink.

<Table 13>

| | |
|---|---|
| Medium chain triacylglycerol | 2.0 g |
| Honey | 15.0 g |
| Citric acid | 0.1 g |
| dl-Malic acid | 0.1 g |
| D-Sorbitol solution (70%) | 10.0 g |
| Sodium benzoate | 0.1 g |
| Perfume | appropriate |
| Purified water | to balance for a total amount of 100 g |

[Example 14: Cereal food]

By the combination ratio shown in Table 14, materials were mixed, water was added thereto, then a mixture was molded and dried with heating in a oven to afford a spherically shaped cereal food.

<Table 14>

| | |
|---|---|
| Medium chain triacylglycerol | 15.0 g |
| Wheat | 30.0 g |
| Defatted soybean | 18.5 g |
| Wheat bran | 15.0 g |
| Wheat germ | 11.5 g |
| Granulated sugar | 10.0 g |

[Example 15: Edible blended fat and oil]

By the combination ratio shown in Table 15, dissolution was performed using an agitator to produce an edible blended fat and oil.

<Table 15>

| | |
|---|---|
| Medium chain triacylglycerol | 20.0 g |
| Soybean oil | 80.0 g |

(Example 16: Margarine]

Raw materials in Table 16 were mixed by a standard method, and kneaded with rapid cooling using a combinator to afford margarine.

<Table 16>

| | |
|---|---|
| Rapeseed oil | 22.0 g |
| Medium chain triacylglycerol | 20.0 g |
| Rapeseed hardened oil | 42.0 g |
| Water | 14.0 g |
| Salt | 0.5 g |
| Lecithin | 0.5 g |
| Monoglyceride | 0.4 g |

<Table 16> (continued)

| | |
|---|---|
| Perfume | appropriate |
| Carotene | trace |

[Example 17: Dressing]

By the combination ratio in Table 17, first raw materials other than soybean salad oil and medium chain triacylglycerol were put into a vessel capable of being heated equipped with a stirrer, heated until an article temperature reached 90°C with stirring at 100 rpm using a propeller stirrer, and stirred for 25 min while retaining the article temperature at 90°C. Subsequently, after cooling until the article temperature was lowered to 20°C, the soybean oil and the medium chain triacylglycerol were combined to afford dressing.

<Table 17>

| | |
|---|---|
| Water | 46.6 g |
| Xanthane gum | 0.1 g |
| Fructose glucose liquid sugar | 5.0 g |
| Salt | 5.0 g |
| MSG | 0.3 g |
| Rice vinegar | 10.0 g |
| Pepper | appropriate |
| Medium chain triacylglycerol | 10.0 g |
| Soybean salad oil | 22.0 g |

[Example 18: Mayonnaise]

By the combination ratio in Table 18, first raw materials other than soybean salad oil, medium chain triacylglycerol and salted egg yolk were heated to 90°C with mixing and stirring, and stirred for 25 min while retaining the temperature at 90°C. After cooling to 20°C, the soybean salad oil, the medium chain triacylglycerol and the salted egg yolk were combined, and mixed under reduced pressure to afford mayonnaise.

<Table 18>

| | |
|---|---|
| Soybean salad oil | 45.0 g |
| Medium chain triacylglycerol | 30.0 g |
| Water | 8.4 g |
| Sugar | 1.0 g |
| Sodium glutamate | 0.3 g |
| Powdered mustard | 0.3 g |
| Salt | 1.0 g |
| Rice vinegar (acid degree: 10%) | 4.0 g |
| Salted egg yolk | 10.0 g |

[Production Example 1]

After 80 parts by mass of rapeseed refined oil (supplied by Nisshin Oil Mills, Ltd.) and 20 parts by mass of MCT where constituent fatty acids were caprylic acid/capric acid = 3/1 at a mass ratio were mixed, a mixture was stirred at 120°C under reduced pressure to perform deaeration and dehydration. Thereto, 0.1 parts by mass of sodium methylate was added as a catalyst, and random ester exchange reaction was performed at 120°C for 30 min. A reaction product was washed with water and dried by common procedures. Subsequently, decolorization and deodorization were performed to afford a fat and oil composition A.

<Table 19>

| [Margarine] | |
|---|---|
| Fat and oil composition A | 39.0 g |

<Table 19> (continued)

| [Margarine] | |
|---|---|
| Rapeseed hardened oil | 42.0 g |
| Water | 17.0 g |
| Salt | 0.5 g |
| Lecithin | 0.5 g |
| Monoglyceride | 0.4 g |
| Perfume | appropriate |
| Carotene | trace |

Raw materials shown in the above Table 19 were mixed by a standard method, and kneaded with rapid cooling to afford the margarine.

<Table 20>

| [Dressing] | |
|---|---|
| Water | 46.6 g |
| Xanthane gum | 0.1 g |
| Fructose glucose liquid sugar | 5.0 g |
| Salt | 5.0 g |
| MSG | 0.3 g |
| Rice vinegar (acid degree: 10%) | 10.0 g |
| Pepper | appropriate |
| Fat and oil composition A | 32.0 g |

By the combination ratio shown in the above Table 20, first, raw materials other than the fat and oil composition A were put into a vessel capable of being heated equipped with a stirrer, heated until an article temperature reached 90°C with stirring at 100 rpm using a propeller stirrer, and stirred for 25 min while retaining the article temperature at 90°C. Subsequently, after cooling until the article temperature was lowered to 20°C, the fat and oil composition A was combined to afford the dressing.

<Table 21>

| [Mayonnaise] | |
|---|---|
| Fat and oil composition A | 75.0 g |
| Water | 8.4 g |
| Sugar | 1.0 g |
| Sodium glutamate | 0.3 g |
| Powdered mustard | 0.3 g |
| Salt | 1.0 g |
| Rice vinegar | 4.0 g |
| Salted egg yolk | 10.0 g |

By the combination ratio in the above Table 21, first raw materials other than the fat and oil composition A and salted egg yolk were heated to 90°C with mixing and stirring, and stirred for 25 min while retaining the temperature at 90°C. After cooling to 20°C, the fat and oil composition A and the salted egg yolk were combined, and mixed under reduced pressure to afford the mayonnaise.

By the use of the body temperature elevating agent of the invention, it is possible to obtain anti-obesity effects and the like, and also obtain an effect of body temperature elevation. Furthermore, by enforcing the functions, it is possible to obtain effective improving agents for poor blood circulation. Also by exploiting this as one function, it is possible to use for various intended uses, and design preparations, medicines for external use, foods and drinks, and the like for various people.

## Claims

1. A body temperature elevating agent which is comprised of medium chain fatty acid and/or glycerin fatty acid ester including the medium chain fatty acid as an active ingredient.

2. The body temperature elevating agent according to Claim 1, wherein a carbon number of fatty acid residues of the medium chain fatty acid and/or the glycerin fatty acid ester including the medium chain fatty is from 6 to 12.

3. The body temperature elevating agent according to Claim 1 or 2, wherein the fatty acid is comprised of one or two or more types selected from the group consisting of caproic acid, caprylic acid, capric acid, and lauric acid as an active ingredient(s).

4. A body temperature elevating agent which is comprised of medium chain fatty acid glycerin fatty acid triester as an active ingredient.

5. The body temperature elevating agent according to Claim 4, wherein a carbon number of fatty acid residues of the medium chain fatty acid and/or the glycerin fatty acid ester including the medium chain fatty is from 6 to 12.

6. The body temperature elevating agent according to Claim 4 or 5, wherein the fatty acid comprised of one or two or more types selected from the group consisting of caproic acid, caprylic acid, capric acid, and lauric acid an active ingredient(s).

7. The body temperature elevating agent according to any one of Claims 1 to 6, wherein further containing one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

8. The body temperature elevating agent according to any one of Claims 1 to 7, wherein further containing one or two or more selected from the group consisting of vitamin B group, vitamin E, lecithin, minerals and herbs.

9. An agent for poor blood circulation, wherein containing (A) medium chain fatty acid and/or glycerin fatty acid ester including the medium chain fatty acid, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

10. An agent for poor blood circulation, wherein containing (A) medium chain triacylglycerol, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides.

11. An agent for women wherein containing (A) medium chain fatty acid and/or glycerin fatty acid ester including the medium chain fatty acid, (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides, and (C) one or two or more selected from the group consisting of γ-linolenic acid, dihome-γ-linolenic acid, calcium, magnesium, zinc, iron, vitamin B6, vitamin E, St. John's wort and extracts thereof, safflower and extracts thereof, saffron and extracts thereof, and lemon balm and extracts thereof.

12. An agent for women wherein containing (A) medium chain triacylglycerol, and (B) one or two or more selected from the group consisting of a highly unsaturated fatty acid or a fatty acid ester including this, conjugated linoleic acid or a fatty acid ester including this, capsaicins, thiazolidine dione derivatives, genus Cirsium plants, black five grains, and pituitary adenylate cyclase activation peptides, and (C) one or two or more selected from the group consisting of γ-linolenic acid, dihome-γ-linolenic acid, calcium, magnesium, zinc, iron, vitamin B6, vitamin E, St. John's wort and extracts thereof, safflower and extracts thereof, saffron and extracts thereof, and lemon balm and extracts thereof.

13. A fat and oil composition for body temperature elevation, which is comprised of medium chain fatty acid and/or

glycerin fatty acid ester including the medium chain fatty acid as an active ingredient.

**14.** A fat and oil composition for body temperature elevation, which is comprised of medium chain fatty acid glycerin fatty acid triester as an active ingredient.

**15.** A food and drink for body temperature elevation which contains medium chain fatty acid and/or glycerin fatty acid ester including the medium chain fatty acid.

**16.** A food and drink for body temperature elevation which contains medium chain fatty acid glycerin fatty acid triester.

**17.** A body temperature elevating agent raw material wherein containing medium chain fatty acid and/or glycerin fatty acid ester including the medium chain fatty acid.

**18.** A body temperature elevating agent raw material wherein containing medium chain triacylglycerol.

**19.** A method for using medium chain fatty acid and/or glycerin fatty acid ester including the medium chain fatty acid as a body temperature elevating agent.

**20.** A method for using medium chain triacylglycerol as a body temperature elevating agent.

**21.** Use of medium chain fatty acid and/or glycerin fatty acid ester including the medium chain fatty acid for the production of a body temperature elevating agent.

# Fig. 1A

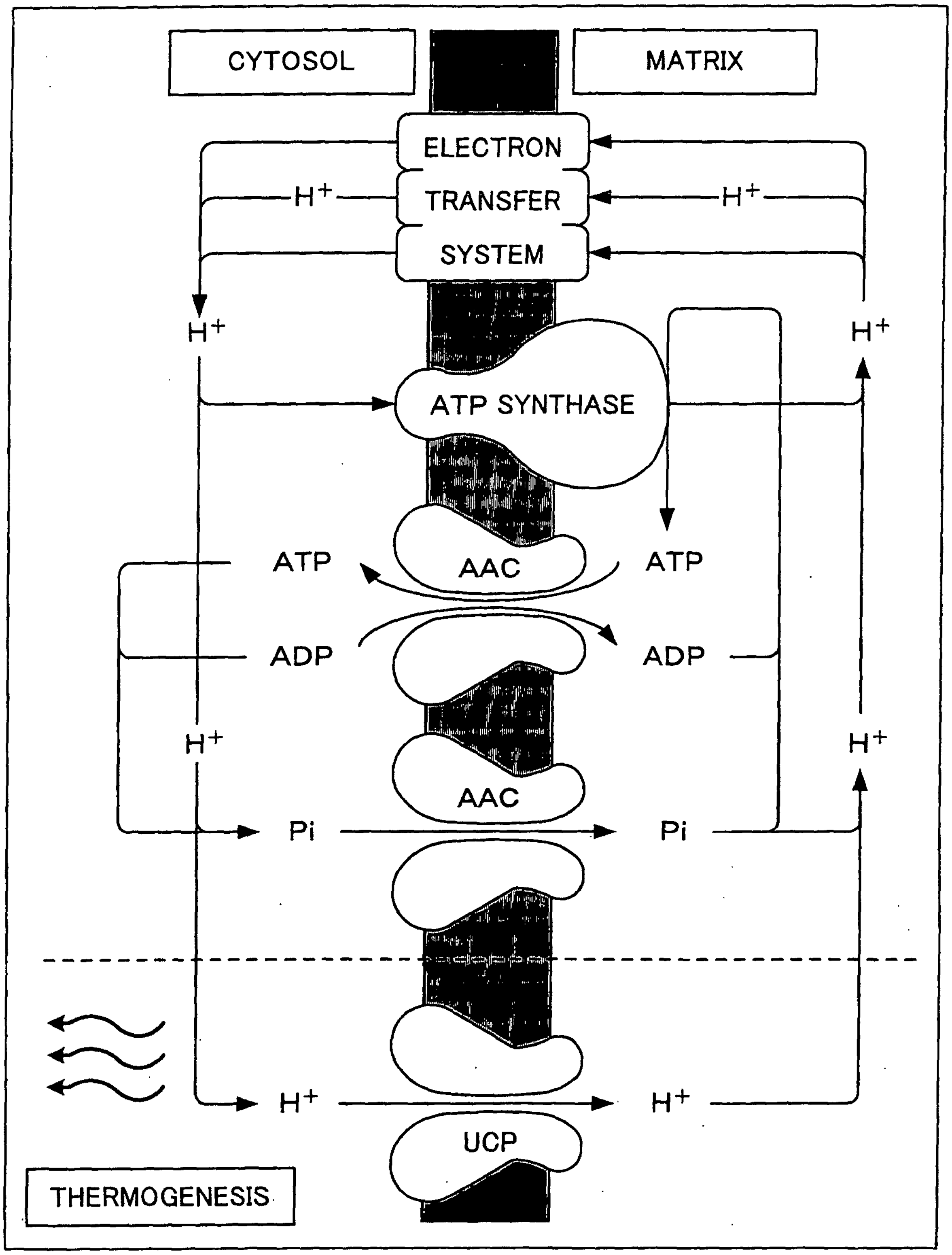
CYTOSOL
MATRIX
ELECTRON
TRANSFER
SYSTEM
H+
H+
H+
H+
ATP SYNTHASE
ATP
AAC
ATP
ADP
ADP
H+
H+
AAC
Pi
Pi
H+
H+
UCP
THERMOGENESIS

# Fig. 1B

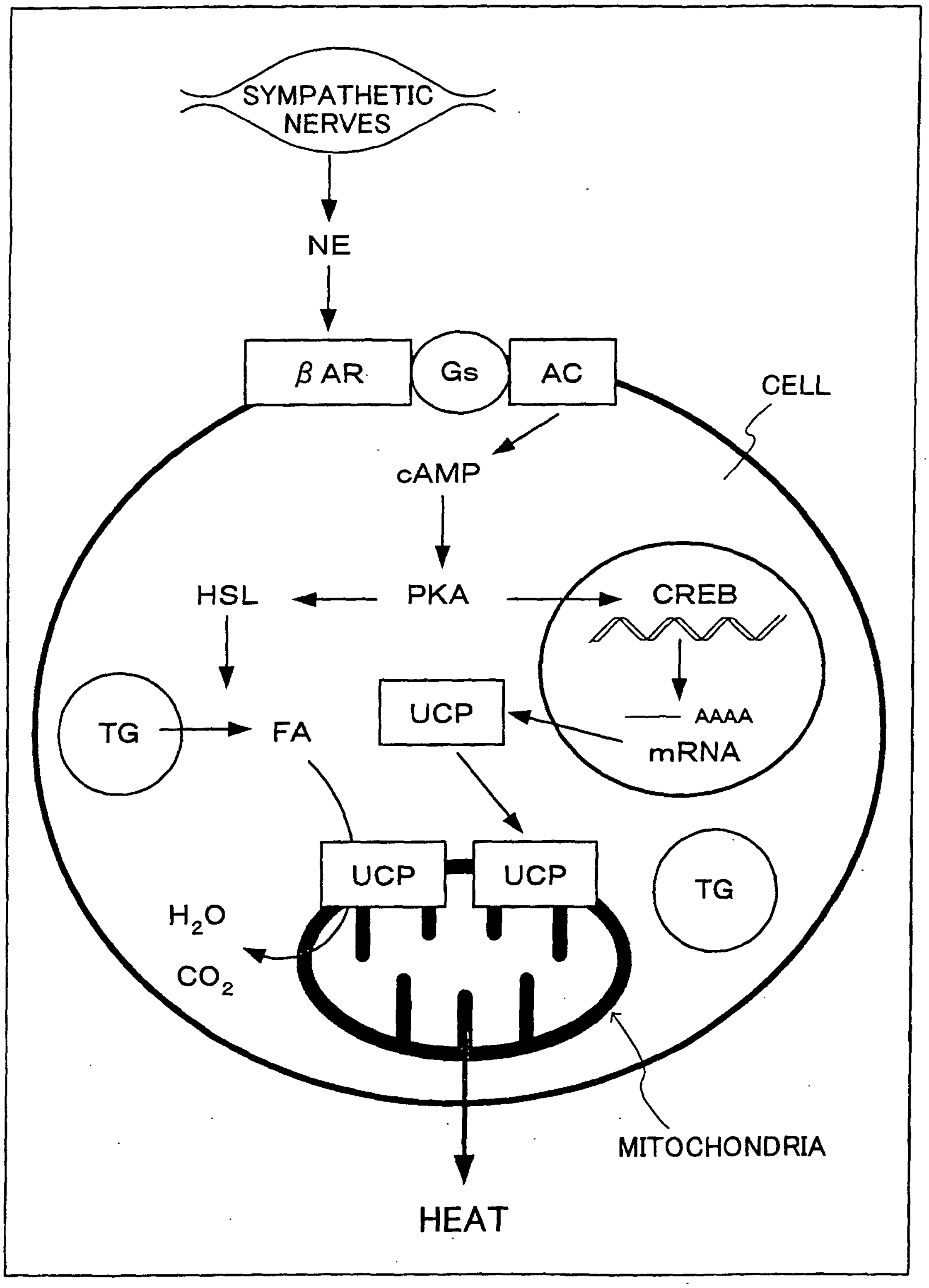
SYMPATHETIC NERVES
NE
β AR
Gs
AC
CELL
cAMP
PKA
HSL
CREB
TG
FA
UCP
AAAA
mRNA
UCP
UCP
TG
$H_2O$
$CO_2$
MITOCHONDRIA
HEAT

**INTERNATIONAL SEARCH REPORT**

International application No.
PCT/JP03/02442

A. CLASSIFICATION OF SUBJECT MATTER
Int.Cl[7] A61K31/23, 31/165, 31/20, 31/355, 31/426, 31/4415, 31/661, 33/06, 33/26, 33/30, 35/78, 38/16, A61P3/04, 3/06, 3/10, 9/00, 43/00, A23D9/00

According to International Patent Classification (IPC) or to both national classification and IPC

B. FIELDS SEARCHED

Minimum documentation searched (classification system followed by classification symbols)
Int.Cl[7] A61K31/23, 31/165, 31/20, 31/355, 31/426, 31/4415, 31/661, 33/06, 33/26, 33/30, 35/78, 38/16, A61P3/04, 3/06, 3/10, 9/00, 43/00, A23D9/00

Documentation searched other than minimum documentation to the extent that such documents are included in the fields searched

Electronic data base consulted during the international search (name of data base and, where practicable, search terms used)
CAPLUS(STN), REGISTRY(STN), Medline(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2002-29967 A (The Nisshin Oil Mills, Ltd.), 29 January, 2002 (29.01.02), Claims; Par. Nos. [0017], [0059], [0068]; table 1 (Family: none) | 1-4,8,13-18, 21<br>7,9-12<br>5,6 |
| X<br>Y | JP 2001-64171 A (Kao Corp.), 13 March, 2001 (13.03.01), Claims; Par. Nos. [0006], [0009], [0011] (Family: none) | 1-3,13,15, 17,21<br>7,9,11 |
| Y | JP 2001-139485 A (Kao Corp.), 22 May, 2001 (22.05.01), Claims (Family: none) | 7,9-12 |

[x] Further documents are listed in the continuation of Box C.  [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 April, 2003 (02.04.03) | 15 April, 2003 (15.04.03) |
| Name and mailing address of the ISA/ Japanese Patent Office | Authorized officer |
| Facsimile No. | Telephone No. |

Form PCT/ISA/210 (second sheet) (July 1998)

INTERNATIONAL SEARCH REPORT

International application No.
PCT/JP03/02442

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2001-81026 A (Director General of National Institute of Health and Nutrition for Ministry of Health and Welfare), 27 March, 2001 (27.03.01), Claims (Family: none) | 7,9-12 |
| X | GB 2084172 A (Roussel Uclaf), 07 April, 1982 (07.04.82), Claims & JP 57-91915 A & US 4407821 A | 15-18 |
| P,X | JP 2002-302441 A (The Nisshin Oil Mills, Ltd.), 18 October, 2002 (18.10.02), Claims (Family: none) | 1-18,21 |

Form PCT/ISA/210 (continuation of second sheet) (July 1998)

**INTERNATIONAL SEARCH REPORT**

International application No.
PCT/JP03/02442

**Box I Observations where certain claims were found unsearchable (Continuation of item 2 of first sheet)**

This international search report has not been established in respect of certain claims under Article 17(2)(a) for the following reasons:

1. [x] Claims Nos.: 19, 20
   because they relate to subject matter not required to be searched by this Authority, namely:
   Claims 19, 20 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.
2. [ ] Claims Nos.:
   because they relate to parts of the international application that do not comply with the prescribed requirements to such an extent that no meaningful international search can be carried out, specifically:
3. [ ] Claims Nos.:
   because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)